# EUROPEAN PATENT APPLICATION

(11) **EP 1 175 893 A2**
(43) Date of publication of application: **30.01.2002**
(21) Application number: 01116743.4
(22) Date of filing: 19.07.2001
(51) Int. Cl.: A61K 7/13

(54) **Hair dye composition**

(30) Priority: 25.07.2000 JP 2000223693
(71) Applicant: Kao Corporation, Tokyo 103-8210 (JP)
(72) Inventor: Oshika, Masato, Kao Corporation Research Lab., Tokyo 131-8501 (JP); Miyabe, Hajime, Kao Corporation Research Lab., Tokyo 131-8501 (JP)
(74) Representative: HOFFMANN - EITLE

(57) **Abstract**

Provided is a hair dye composition containing (A) a cationic direct dye having a triarylmethane structure and (B) an organic solvent represented by any one of the following formulas (1), (2), (3), (4) and (5):

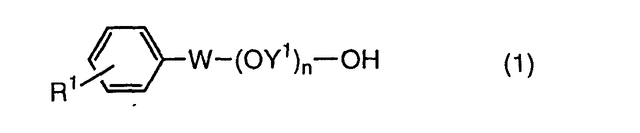

R²―(OY²)ₘ―OH (2)

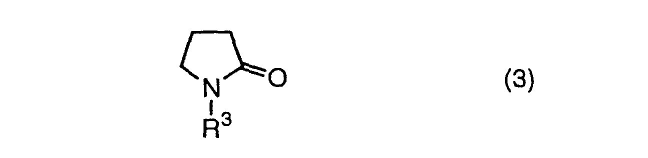

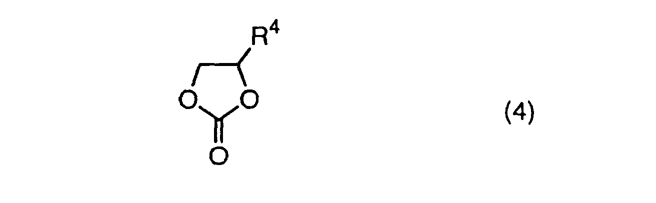

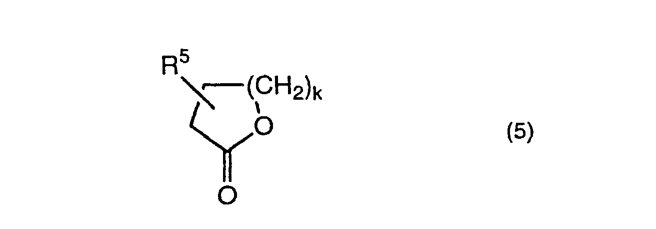

[wherein, R¹ represents a hydrogen atom, a C₁₋₄ alkyl group or a C₁₋₄ alkoxy group, W represents a single bond, a methylene group, or a C₂₋₄ alkylene group, Y¹ and Y² each represents a C₂₋₄ alkylene group, n and m each stands for an integer of 0 to 3, R² represents a C₄₋₇ alkylene group, R³ represents a C₁₋₄ alkyl group, R⁴ represents a hydrogen atom or a C₁₋₄ alkyl group, R⁵ represents a hydrogen atom or a methyl group and k stands for 1 or 2].

This hair dye composition is capable of dyeing the hair intensely and uniformly and is excellent in resistance against shampooing and the like after hair dyeing.

## Description

### Technical Field

The present invention relates to a hair dye composition capable of dyeing the hair intensely and uniformly and having excellent resistance against shampooing and the like after hair dyeing.

### Background Art

A direct dye has been used as a main colorant of a semi-permanent dye and it has been demanded to have intense and uniform hair dyeing capacity and have resistance against circumstances which the dyed hair is exposed to such as light, perspiration and shampooing.

Cationic direct dyes having a triarylmethane structure and usable for dyeing of the human hair are conventionally known, but they are not fully satisfactory in coloring strength, uniform dyeing property and resistance against shampooing and the like after hair dyeing.

An object of the present invention is therefore to provide a hair dye composition which contains a cationic direct dye having a triarylmethane structure, is capable of dyeing the hair intensely and uniformly and has excellent resistance against shampooing and the like after hair dyeing.

### Disclosure of the Invention

The present inventors have found that a cationic direct dye having a triarylmethane structure is markedly improved in coloring strength, uniform dyeing property and resistance against shampooing and the like after hair dyeing when used in combination with a specific organic solvent.

The present invention therefore provides a hair dye composition comprising the following components (A) and (B):
(A) a cationic direct dye having a triarylmethane structure, and
(B) an organic solvent represented by any one of the following formulas (1), (2), (3), (4) and (5): 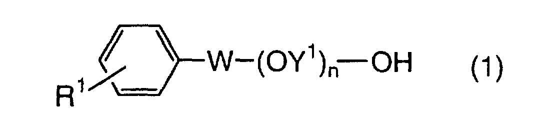 [wherein, R¹ represents a hydrogen atom, a linear or branched C₁₋₄ alkyl group or a linear or branched C₁₋₄ alkoxy group, W represents a single bond, a methylene group, or a linear or branched C₂₋₄ alkylene group, Y¹ represents a linear or branched C₂₋₄ alkylene group and n stands for an integer of 0 to 3].

   R²―(OY²)ₘ―OH (2)

   [wherein, R² represents a linear, branched or cyclic C₄₋₇ alkyl group, Y² represents a linear or branched C₂₋₄ alkylene group and m stands for an integer of 0 to 3]. 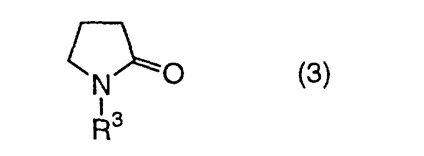 [wherein, R³ represents a linear or branched C₁₋₄ alkyl group]. 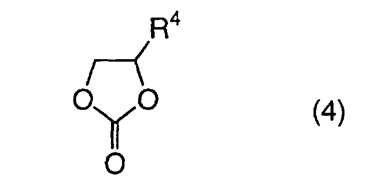 [wherein, R⁴ represents a hydrogen atom or a linear or branched C₁₋₄ alkyl group]. 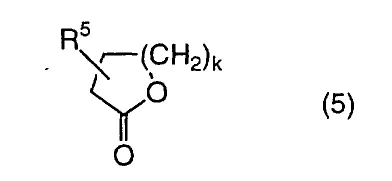 [wherein, R⁵ represents a hydrogen atom or a methyl group and k stands for 1 or 2].

The present invention also provides a hair dyeing method, comprising applying the above-described hair dye composition to the hair.

### Best Mode for Carrying out the Invention

The term "cationic direct dye having a triarylmethane structure" as component A means a cationic direct dye having a triarylmethane structure but not having an acid group such as sulfonic acid group. It does not embrace a cationic direct dye having a xanthene structure or acridine structure. Examples of the cationic direct dye having a triarylmethane structure include C.I. Basic Blue 1, C.I. Basic Blue 2, C.I. Basic Blue 5, C.I. Basic Blue 7, C.I. Basic Blue 8, C.I. Basic Blue 11, C.I. Basic Blue 15, C.I. Basic Blue 18, C.I. Basic Blue 20, C.I. Basic Blue 23, C.I. Basic Blue 26, C. I. Basic Blue 55, C.I. Basic Blue 81, C.I. Basic Green 1, C.I. Basic Green 4, C.I. Basic Red 9, C.I. Basic Violet 1, C.I. Basic Violet 2, C.I. Basic Violet 3, C.I. Basic Violet 4, C.I. Basic Violet 13, C.I. Basic Violet 14, and C.I. Basic Violet 23.

One or more of these cationic direct dyes having a triarylmethane structure can be used as the component (A). Its content is preferably 0.001 to 5 wt.%, more preferably 0.005 to 3 wt.%, especially 0.01 to 1 wt.% based on the whole composition.

Examples of the linear or branched C₁₋₄ alkyl group represented by R¹, R³ or R⁴ in each of the formulas (1) to (5) representing the organic solvent of the component (B) include methyl, ethyl, propyl, isopropyl, butyl, isobutyl, sec-butyl and tert-butyl groups. Examples of the linear or branched C₁₋₄ alkoxy group represented by R¹ include methoxy, ethoxy, propoxy, isopropoxy and butoxy groups. Examples of the linear, branched or cyclic C₄₋₇ alkyl group represented by R² include butyl, pentyl, hexyl, heptyl, isobutyl, cyclopentyl and cyclohexyl groups. Examples of the linear or branched C₂₋₄ alkylene group represented by X, Y¹ or Y² include ethylene, trimethylene, propylene and tetramethylene groups.

Examples of the organic solvent (1) include 2-phenoxyethanol, benzyl alcohol, 2-phenylethanol and 2-benzyloxyethanol, those of the organic solvent (2) include n-butanol, ethylene glycol mono-n-butyl ether and diethylene glycol mono-n-butyl ether, those of the organic solvent (3) include N-methylpyrrolidone and N-ethylpyrrolidone, those of the organic solvent (4) include ethylene carbonate and propylene carbonate, and those of the organic solvent (5) include γ-butyrolactone and γ-valerolactone. Of these, the organic solvent (1), particularly, benzyl alcohol and 2-benzyloxyethanol are preferred.

One or more of these organic solvents may be used as the component (B). Its content is preferably 0.5 to 50 wt.%, more preferably 1 to 45 wt.%, especially 2 to 40 wt.%.

To the hair dye composition of the present invention, a cationic direct dye represented by the following formula (6): 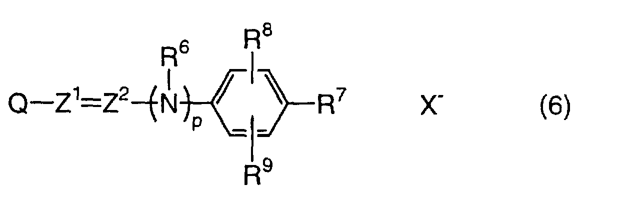 [wherein, Q represents any one of the following groups (q1), (q2) and (q3): 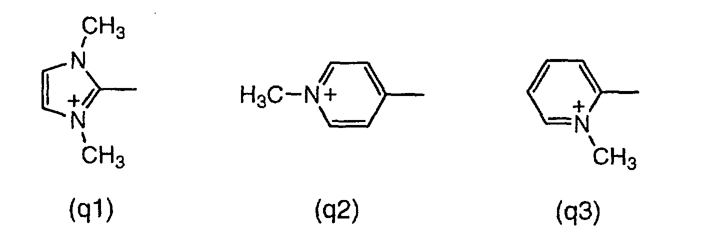 Z¹ and Z² each independently represents a nitrogen atom or a methine group, R⁶ represents a hydrogen atom or a linear or branched C₁₋₄ alkyl group or forms, together with the adjacent nitrogen atom and the carbon atom of the benzene ring, a heterocycle, R⁷ represents a hydrogen atom, a linear or branched C₁₋₄ alkoxy group or a group -NR¹⁰R¹¹ (in which R¹⁰ and R¹¹ each independently represents a hydrogen atom or a linear or branched C₁₋₄ alkyl group or forms, together with the adjacent nitrogen atom and the carbon atom of the benzene ring, a heterocycle), R⁸ and R⁹ each independently represents a hydrogen atom, a halogen atom, a linear or branched C₁₋₄ alkyl group or a linear or branched C₁₋₄ alkoxy group, p stands for 0 or 1 and X⁻ represents an anion] may be incorporated as component (C).

Examples of the linear or branched C₁₋₄ alkyl group represented by R⁶, R⁸, R⁹, R¹⁰ or R¹¹ in the formula (6) include methyl, ethyl, propyl, isopropyl, butyl, isobutyl, sec-butyl and tert-butyl groups. Examples of the heterocycle formed by R⁶, R¹⁰ or R¹¹, together with the adjacent nitrogen atom and the carbon atom of the benzene ring include morpholine, pyrrolidine, piperidine and piperazine rings. Examples of the linear or branched C₁₋₄ alkoxy group represented by R⁷, R⁸ or R⁹ include methoxy, ethoxy, propoxy, isopropoxy and butoxy groups. Examples of the anion represented by X⁻ include chloride ions, bromide ions, iodide ions, trichlorozincic acid ions, tetrachlorozincic acid ions, sulfuric acid ions, hydrosulfuric acid ions, methyl sulfate ions, phosphoric acid ions, formic acid ions and acetic acid ions.

The color brought by the cationic direct dye of the component (A) is limited to a cold color, mainly, blue color, while the cationic direct dye (6) as the component (C) imparts a warm color such as yellow, orange and red and has excellent dyeing property. Use of them in combination makes it possible to dye the human hair into a dark brown to black color excellent in coloring strength, uniform dyeing property and resistance against shampooing and the like after hair dyeing.

The cationic direct dye (6) as component (C) is a known dye described in the pamphlet of International Patent Laid-Open No. 95/01772 or International Patent Laid-Open No. 95-15144. Preferred examples of it include:

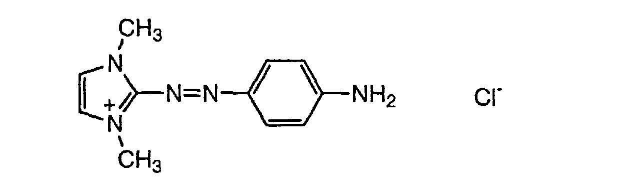

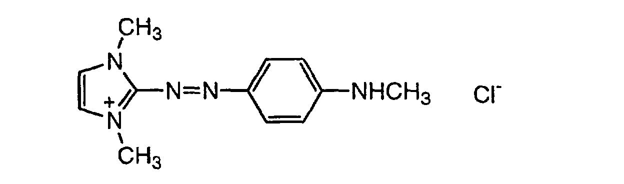

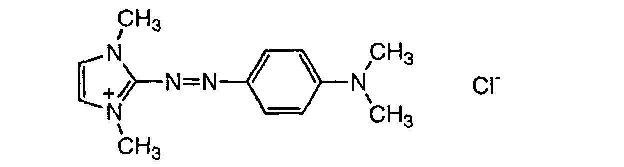

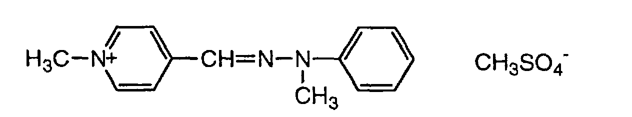

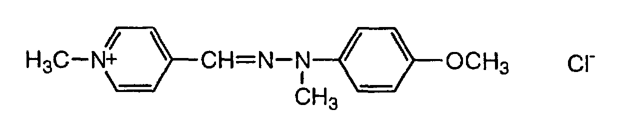

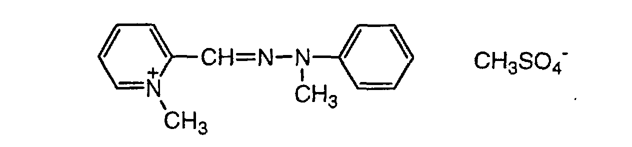

One or more of these cationic direct dyes (6) can be used as component (C). Its content is preferably 0.001 to 5 wt.%, more preferably 0.005 to 3 wt.%, especially 0.01 to 1 wt.% based on the whole composition.

For the hair dye composition of the present invention, water and/or an organic solvent is used as a medium. Examples of the organic solvent include lower alkanols such as ethanol and 2-propanol, polyols such as propylene glycol, 1,3-butanediol and diethylene glycol, cellosolves such as ethyl cellosolve and carbitols such as ethyl carbitol.

The hair dye composition of the present invention is preferred to have a pH of at least 6, more preferably at least 8, especially 8 to 11 from the viewpoints of dyeing property and fastness. Its pH can be adjusted by a pH regulator or the like. Examples of the pH regulator include inorganic acids such as hydrochloric acid and phosphoric acid, organic acids such as citric acid, glycolic acid and lactic acid, hydrochlorides such as ammonium chloride, organic amines such as monoethanolamine and 2-amino-2-methylpropanol, bicarbonates such as sodium bicarbonate and ammonium bicarbonate, carbonates such as ammonium carbonate and sodium carbonate, phosphates such as monopotassium dihydrogenphosphate and disodium monohydrogenphosphate, hydroxides such as sodium hydroxide and aqueous ammonia.

The hair dye composition of the present invention is able to have improved hair dyeing effects and improved hair conditioning effects after dyeing by the incorporation therein a cationic surfactant, cationic polymer or silicone. Examples of the cationic surfactant include those represented by the following formula (7): 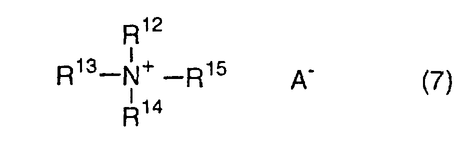 [wherein, one or two of R¹², R¹³, R¹⁴ and R¹⁵ each represents a linear or branched C₈₋₃₀ alkyl group or alkenyl group and the remaining two or three each represents a C₁₋₃ alkyl group or hydroxyalkyl group or benzyl group, and A⁻ represents an anion].

Specific examples include lauryltrimethylammonium chloride, lauryltrimethylammonium bromide, cetyltrimethylammonium chloride, cetyltrimethylammonium bromide, stearyltrimethylammonium chloride, stearyltrimethylammonium bromide, dicetyldimethylammonium chloride, distearyldimethylammonium chloride, dicocoyldimethylammonium chloride and benzalconium chloride. As the cationic surfactant, one or more of the above-exemplified ones can be used. For bringing about sufficient effects, it is incorporated preferably in an amount of 0.1 to 5 wt.%, especially 0.5 to 2 wt.% based on the whole composition.

Examples of the cationic polymer include cationated cellulose derivatives, cationated guar gum derivatives, cationated polysaccharides, diallyl dialkyl quaternary ammonium salt derivatives, and cationated polyvinyl pyrrolidone derivatives. Of these, diallyl dialkyl quaternary ammonium salt derivatives are preferred. As the cationic polymer, one or more of the above-exemplified ones can be used. For bringing about sufficient effects, it is incorporated preferably in an amount of 0.1 to 2 wt.%, especially 0.3 to 1 wt.% based on the whole composition. Exemplary silicones include dimethylpolysiloxane, methylphenylpolysoloxane, polyether-modified silicone, amino-modified silicone and poly(N-acylalkyleneimine)modified silicone. As the silicone, one or more of the above-exemplified ones can be used. For bringing about sufficient effects, it is incorporated preferably in an amount of 0.01 to 3 wt.%, especially 0.1 to 2 wt.% based on the whole composition.

The hair dye composition of the present invention is preferred to have a viscosity, upon application to the hair, of 500 to 100000 mPa·s, because such a viscosity facilitates application of it to the hair. Thickening can be conducted by gelation using a thickening agent, or by forming the composition in the form of an emulsion or emulsified cream. Examples of the thickening agent include natural high molecules such as guar gum, xanthane gum and cellulose, and hydroxyalkylated derivatives, carboxyalkylated derivatives or cationated derivatives (the above-described cationic polymers) thereof.

In addition to them, the hair dye composition of the present invention can contain a direct dye other than components (A) and (C), an autoxidation dye such as 5,6-dihydroxyindole or 5,6-dihydroxyindoline, anti-oxidant, sequestering agent, penetrant, buffer, surfactant, ultraviolet absorber, humectant, antiseptic, sterilizer, dispersant, film forming agent, oil agent, pigment and propellant. As the form of the hair dye composition of the present invention, any one suited for hair dyeing can be used. Examples include powder, transparent liquid, emulsion, cream, gel, paste, aerosol, and aerosol foam.

For dyeing the hair with the hair dye composition of the present invention, it is recommended to apply at least one of the invention compositions to the hair, allow it to stand for time (1 to 60 minutes, especially 5 to 40 minutes) enough to impart the hair with the desired color tone, rinse the hair, and, if necessary after shampooing and rinsing, dry the dyed hair.

### -Examples-

### Example 1

Hair dyes as shown in Table 1 were prepared and their dyeing power was evaluated.

### (Evaluation method)

From the blonde hair of Americans, five hair bundles each 15 cm long and 20 g in weight were prepared. To each of the hair bundles, 15 g of each of the hair dyes prepared as Invention Products 1 and 2 and Comparative Products 1 to 3 was applied all over the hair bundle. After the resulting hair bundles were allowed to stand in a thermostat of 30°C for 20 minutes, they were rinsed with warm water of 36°C sufficiently and then dried. Based on the observation by a panel of 10 experts, the hair dyeing power of each of the hair dyes was evaluated in accordance with the below-described standards. Total points are indicated in Table 1.

### (Evaluation standards)

The hair bundle seems to be tinted intensely: 3
The hair bundle seems to be tinted slightly intensely: 2
The hair bundle seems to be tinted only lightly: 1
The hair bundle does not seem to be tinted: 0

**Table 1**

| (wt.%) | Invention product | | Comparative Product | | |
|---|---|---|---|---|---|
| | 1 | 2 | 1 | 2 | 3 |
| C.l. Basic Blue 7 [Component (A)] | 0.1 | - | 0.1 | - | - |
| C.l. Basic Blue 26 [Component (A)] | - | 0.1 | - | 0.1 | - |
| C.l. Acid Blue 9 | - | - | - | - | 0.1 |
| Benzyl alcohol | 4 | 4 | - | - | 4 |
| Ethanol | 15 | | | | |
| Hydroxypropyl guar gum | 1.5 | | | | |
| Monoethanolamine | 0.15 | | | | |
| Phosphoric acid | Amount to adjust pH to 9 | | | | |
| Water | Balance | | | | |
| Evaluation of dyeing power | 30 | 30 | 10 | 10 | 10 |

### Example 2

Hair dyes having a composition as described below in Table 2 were prepared.

**Table 2**

| (wt%) | Invention Products | | | | | |
|---|---|---|---|---|---|---|
| | 3 | 4 | 5 | 6 | 7 | 8 |
| C.l. Basic Blue 7 [Component (A)] | 0.1 | 0.05 | - | 0.1 | - | 0.05 |
| C.l. Basic Violet 14 [Component (A)] | - | - | 0.02 | - | 0.02 | - |
| C.l. Basic Red 51 [Component (C)] | - | 0.01 | - | - | - | 0.01 |
| C.l. Basic Orange 31 [Component (C)] | 0.1 | - | 0.05 | 0.1 | 0.05 | - |
| C.l. Basic Yellow 87 [Component (C)] | - | 0.05 | 0.05 | - | 0.05 | 0.05 |
| Benzyl alcohol | 10 | - | - | - | - | - |
| 2-Benzyloxyethanol | - | 10 | - | - | - | - |
| Ethylene glycol mono-n-butyl ether | - | - | 10 | - | - | - |
| N-methylpyrrolidone | - | - | - | 10 | - | - |
| Propylene carbonate | - | - | - | - | 10 | - |
| γ-Butyrolactone | - | - | - | - | - | 10 |
| Ethanol | 10 | | | | | |
| Propylene glycol | 5 | | | | | |
| Hydroxypropyl guar gum | 1.5 | | | | | |
| "Gafquat 734" ^{*1} | 1 | - | - | 1 | - | - |
| "Catinal LC100" ^{*2} | - | 1 | 1 | - | 1 | 1 |
| Polyether-modified silicone "KF6005 ^{*3} | 1 | | | | | |
| Amodimethicone SM8702C ^{*4} | 1 | | | | | |
| Monoethanolamine | 0.1 | | | | 0.01 | |
| Phosphoric acid | Amount to adjust pH 9 | | | | Amount to adjust pH7 | |
| Water | Balance | | | | | |

| | | | | | | |
|---|---|---|---|---|---|---|
| *1: Copolymer of vinylpyrrolidone and N,N-dimethylaminoethyl methacrylate in the form of a diethyl sulfate salt solution; product of ISP Japan) | | | | | | |
| *2: O-[2-Hydroxy-3-(trimethylammonio)propyl]hydroxyethyl cellulose chloride; product of Toho Chemical Industry | | | | | | |
| *3: Copolymer of polyoxyethylene and methylpolysiloxane; product of Shin-Etsu Chemical | | | | | | |
| *4: Copolymer of aminoethylaminopropylsiloxane and dfimethylsiloxane in the form of emulsion; product of Dow Corning Toray Silicone | | | | | | |

Rightly after shampooing, the invention product 3 was applied to the light-brown, damp-dry hair (gray hair ratio: 70%). The resulting hair was allowed to stand for 5 minutes at room temperature, rinsed with warm water and then dried. As a result, the hair was tinted intensely and uniformly with black.

The Invention Products 4 to 8 were each applied to the light-brown hair (gray hair ratio: 70%). The resulting hair was allowed to stand for 15 minutes at room temperature, rinsed with warm water, shampooed, rinsed again and then dried. As a result, the hair dyed with Invention Product 4, 6 or 8 was tinted intensely and uniformly with black, while the hair dyed with Invention Product 5 or 7 was tinted intensely and uniformly with dark brown.

## Claims

1. A hair dye composition comprising the following components (A) and (B):
(A) a cationic direct dye having a triarylmethane structure, and
(B) an organic solvent represented by any one of the following formulas (1), (2), (3), (4) and (5): 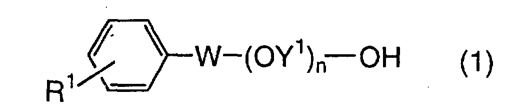 [wherein, R¹ represents a hydrogen atom, a linear or branched C₁₋₄ alkyl group or a linear or branched C₁₋₄ alkoxy group, W represents a single bond, a methylene group, or a linear or branched C₂₋₄ alkylene group, Y¹ represents a linear or branched C₂₋₄ alkylene group and n stands for an integer of 0 to 3].
R²―(OY²)ₘ―OH (2)
[wherein, R² represents a linear, branched or cyclic C₄₋₇ alkyl group, Y² represents a linear or branched C₂₋₄ alkylene group and m stands for an integer of 0 to 3]. 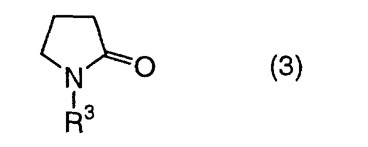 [wherein, R³ represents a linear or branched C₁₋₄ alkyl group]. 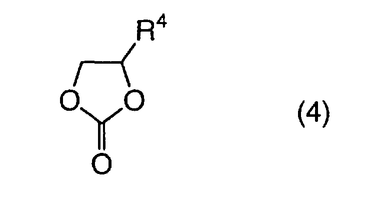 [wherein, R⁴ represents a hydrogen atom or a linear or branched C₁₋₄ alkyl group]. 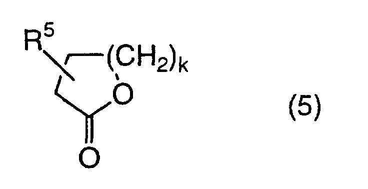 [wherein, R⁵ represents a hydrogen atom or a methyl group and k stands for 1 or 2].

2. A hair dye composition according to claim 1, further comprising the following component (C): 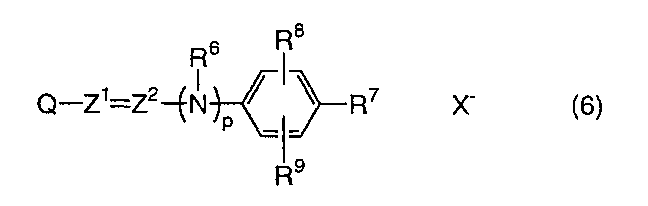 [wherein, Q represents any one of the following groups (q1), (q2) and (q3): 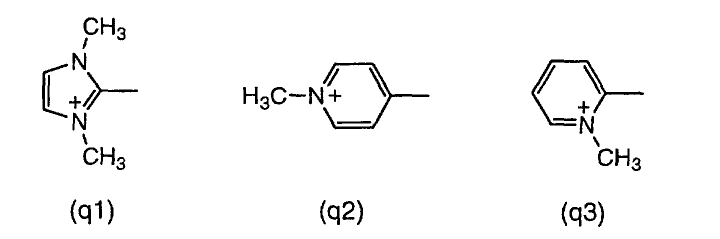 Z¹ and Z² each independently represents a nitrogen atom or a methine group, R⁶ represents a hydrogen atom or a linear or branched C₁₋₄ alkyl group or forms, together with the adjacent nitrogen atom and the carbon atom of the benzene ring, a heterocycle, R⁷ represents a hydrogen atom, a linear or branched C₁₋₄ alkoxy group or a group -NR¹⁰R¹¹ (in which R¹⁰ and R¹¹ each independently represents a hydrogen atom or a linear or branched C₁₋₄ alkyl group or forms, together with the adjacent nitrogen atom or the carbon atom of the benzene ring, a heterocycle), R⁸ and R⁹ each independently represents a hydrogen atom, a halogen atom, a linear or branched C₁₋₄ alkyl group or a linear or branched C₁₋₄ alkoxy group, p stands for 0 or 1 and X⁻ represents an anion].

3. A hair dye composition according to claim 1 or 2, which has a pH of 6 or greater.

4. A hair dyeing method, comprising applying a hair dye composition as claimed in any one of claims 1 to 3 to the hair.
